Europäisches Patentamt

European Patent Office

Office européen des brevets

(19)

(11) Veröffentlichungsnummer: **0 068 086**
**B1**

(12) **EUROPÄISCHE PATENTSCHRIFT**

(45) Veröffentlichungstag der Patentschrift: 11.02.87

(21) Anmeldenummer: 82102970.9

(22) Anmeldetag: 07.04.82

(51) Int. Cl.⁴: **G 01 N 21/88,** G 01 N 17/00,
B 07 C 5/342

(54) **Verfahren zum Erkennen und Ausscheiden gealterter, nicht mehr gebrauchstüchtiger Kunststoffgegenstände.**

(30) Priorität: 03.06.81 DE 3121928

(43) Veröffentlichungstag der Anmeldung:
05.01.83 Patentblatt 83/01

(45) Bekanntmachung des Hinweises auf die Patenterteilung:
11.02.87 Patentblatt 87/07

(84) Benannte Vertragsstaaten:
BE CH FR GB LI LU NL

(56) Entgegenhaltungen:
FR-A-1 501 766

PLASTICS ENGINEERING, Band 28, Juli 1972, Seiten 19-24, F.H. WINSLOW et al.: "Polymers under the weather"

AUTOMATION, Band 17, Nr. 1, Januar 1970, Seiten 53-57, V. HARRIS: "Electro-optics sort products by color"

(73) Patentinhaber: **HÜLS AKTIENGESELLSCHAFT Patentabteilung / PB 15 - Postfach 13 20 D-4370 Marl 1 (DE)**

(72) Erfinder: **Betker, Alexander Romstrasse 38 D-3400 Göttingen (DE)**
Erfinder: **König, Helmut, Dr. Wilhelm-Busch-Strasse 3 D-3406 Bovenden (DE)**
Erfinder: **Peitscher, Günter Alte Strasse 67 D-4370 Marl (DE)**
Erfinder: **Potthoff, Paul Emslandstrasse 3 D-4370 Marl (DE)**

(58) References cited:
**REVUE GENERALE DE L'ELECTRICITE, Band 88, Nr. 9, September 1979, Seiten 685-689, P. LAURENSON et al.: "Photovieillissement et environnement. Nouveau dispositif de photovieillissement accéléré et élaboration d'isolants de couleurs photostables"**

EP 0 068 086 B1

**Beschreibung**

Die Erfindung betrifft ein Verfahren zum Erkennen und Ausscheiden gealterter, nicht mehr gebrauchstüchtiger Kunststoffgegenstände aus dem weiteren Gebrauch.

Die Erfindung bezweckt, kostspielige Folgeschäden zu vermeiden, die durch gealterte, nicht mehr gebrauchstüchtige Kunststoffgegenstände verursacht werden können.

Bekanntlich läuft in (thermoplastischen) Kunststoffgegenständen bei Einwirkung von Lichtstrahlung (im sichtbaren oder unsichtbaren Bereich) in Gegenwart von Luft (Sauerstoff) und Wärme ein—chemischer und physikalischer—Alterungsprozeß ab, und zwar zunächst an der Oberfläche der Gegenstände. Dieser Alterungsprozeß ist wegen des unterschiedlichen Ausmaßes der Einwirkung von Licht, Sauerstoff und Wärme auf den Kunststoffgegenstand unabhängig vom Alter und wenig abhängig von der Gebrauchshäufigkeit der Kunststoffgegenstände; deshalb kann man aus dem Alter der Gegenstände allein nicht auf deren Alterungszustand schließen. Die Alterung führt—ausgehend von der Oberfläche der Gegenstände—zu einer Versprödung des Kunststoffs und damit zur Minderung der Gebrauchstüchtigkeit des Gegenstandes.

Bei Kunststoffgegenständen für wiederholten Gebrauch, z.B. Mehrweg-Flaschentransportkästen aus in der Masse gefärbten Polyolefinen, werden durch eine hinreichend große Alterung Folgeschäden verursacht; diese können sich als erhebliche Störungen bei der automatischen Abfüllung in der Getränkeindustrie äußern oder im Zusammenbrechen von Kastenstapeln oder Palettenstapeln im Lager oder auf dem Transport.

Der Alterungszustand und damit die Gebrauchstüchtigkeit nach einer gewissen Gebrauchsdauer läßt sich nach verschiedenen chemischen, optischen oder mechanischen Verfahren feststellen; diese sind stets aufwendig und meist nicht zerstörungsfrei und damit nur auf einzelne Kunststoffgegenstände anwendbar. Auch die bekannten zerstörungsfreien Verfahren sind bei sehr großen Stückzahlen von Kunststoffgegenständen routinemäßig nicht einsetzbar. Damit fehlt bisher ein zerstörungsfreies Verfahren, mit dem man den Alterungszustand von Kunststoffgegenständen auf einfache Weise feststellen kann, damit man aus einer sehr großen Anzahl gleichartiger Gegenstände diejenigen erkennen und ausscheiden kann, die wegen fortgeschrittener Alterung nicht mehr gebrauchstüchtig sind.

Der Erfindung liegt die Aufgabe zugrunde, den Alterungszustand von Kunststoffgegenständen—unabhängig von ihrem Alter—mittels einer zerstörungsfreien berührungslosen Meßmethode festzustellen zwecks Ausscheiden nicht mehr gebrauchstüchtiger Gegenstände aus einer Vielzahl gleicher Gegenstände.

Die Aufgabe wird erfindungsgemäß gelöst durch

— Beleuchten der Kunststoffgegenstände mit einer Lichtquelle, die Licht im gesamten sichtbaren Spektralbereich aussendet (weißes Licht),

— Messen der Remission an mindestens jeweils einer kritischen Stelle der Oberfläche der Kunststoffgegenstände in einem auf die Farbe der Kunststoffgegenstände abgestimmten Bereich des Spektrums und

— Ausscheiden aller Kunststoffgegenstände, deren Remission außerhalb eines vorgegebenen Sollbereichs liegt.

Als Lichtquellen sind weiße Leuchtstofflampen, Halogenlampen oder Xenon-Hochdrucklampen geeignet; in den Beleuchtungsstrahlengang wird kein Farbfilter eingeschaltet. Der Wellenlängenbereich des von der Oberfläche der Kunstoffgegenstände remittierten Lichtes muß in hohem Anteil in den von der Lichtquelle ausgestrahlten Licht enthalten sein.

Die Remission wird nach einem der bekannten Verfahren unter Verwendung eines handelsüblichen Farbmeßgerätes und unter Beachtung der für optische Untersuchungen notwendigen Sorgfalt gemessen. Aus dem remittierten Licht, dessen Spektralbereich und Intensität von der Farbe des Kunststoffgegenstandes und dem Zustand der Oberfläche abhängig ist, wird ein geeigneter Spektralbereich durch ein Farbfilter im Remissionsstrahlengang ausgefiltert, damit das Verfahren hinreichend empfindlich wird. Die Remission wird bevorzugt in einem Spektralbereich gemessen, der außerhalb des Bereiches der Farbe des Kunststoffgegenstandes liegt.

Der Sollbereich, in welchem die Remission der noch gebrauchstüchtigen Gegenstände liegen muß, richtet sich nach den für den vorliegenden Kunststoffgegenstand spezifischen Anforderungen an die Gebrauchstüchtigkeit.

Das erfindungsgemäße Verfahren beruht auf dem Zusammenhang zwischen Alterungszustand des Kunststoffgegenstandes und Remission der Oberfläche. Das Maß für den Alterungszustand ist die Differenz zwischen den Remissionswerten der Oberfläche eines völlig unbenutzten Kunststoffgegenstandes und des gealterten Kunststoffgegenstandes der gleichen Art und Farbe in einem ausgewählten Wellenlängenbereich bevorzugt außerhalb des Wellenlängenbereichs, in dem der Kunststoffgegenstand die größte Remission zeigt, wobei die Meßbedingungen für beide Gegenstände jeweils gleich sind.

Als kritische Stellen der Oberfläche der Kunststoffgegenstände werden solche Stellen bezeichnet, die bei üblichem Gebrauch der Einwirkung von Licht, Sauerstoff und Wärme sowie mechanischen Zug-, Druck- und Biegebeanspruchungen ausgesetzt sind und keine mechanischen Beschädigungen oder Veränderungen aufweisen (wie Kratzer, Schleif- oder Scheuerspuren, Aufkleber, Klebstoffe, Fremdkörper, Aufdrucke, Prägungen).

Es hat sich als zweckmäßig erwiesen, die Remission an zwei kritischen Stellen der Oberfläche der Kunststoffgegenstände zu messen, die auf zwei etwa senkrecht aufeinander stehenden Außenseiten eines Gegenstandes liegen, die bei bestimmungsgemäßen Einsatz des Gegenstandes der Lichteinwirkung ausgesetzt sind; falls es erforderlich ist, kann die Remission auf mehr als zwei oder auf allen Seiten des Gegenstandes gemessen werden. In diesen Fällen sind mehrere Lichtquellen und jeweils ein Empfänger für jede zu messende Seite des Gegenstandes notwendig.

Ein Kunststoffgegenstand wird ausgeschieden und vom weiteren Gebrauch ausgeschlossen, falls die Remission an einer oder mehr als einer kritischen Stelle außerhalb des vom Kunststoffgegenstand abhängigen Sollbereichs liegt.

Das erfindungsgemäße Verfahren hat folgende Vorteile:

— Es ist von der Schwankung des Pigmentgehaltes der Kunststoffgegenstände praktisch unabhängig.
— Es ist zerstörungsfrei und berührungslos und mit vertretbarem Aufwand verbunden.
— Es läßt sich in bereits bestehende Vorrichtungen zur Handhabung der Kunststoffgegenstände leicht einbeziehen.
— Es läßt den Alterungszustand der Gegenstände erkennen, obwohl eine Vielzahl von Gegenständen gleicher Art visuell praktisch nicht zu unterscheiden sind.
— Es arbeitet automatisch und läßt sich besonders bei sehr großen Stückzahlen von Kunststoffgegenständen kontinuierlich einsetzen.
— Es ist frei von subjektiven Einflüssen.

Das erfindungsgemäße Verfahren ist anwendbar auf in der Masse pigmentierte Kunststoffgegenstände. Die Gegenstände können verschiedene Formen und Farben haben und für verschiedene Zwecke benutzt werden.

Gealterte Mehrweg-Flaschentransportkästen aus Polyolefinen zum Beispiel, die ein zu großes Sicherheitsrisiko für den weiteren Gebrauch darstellen, sind durch folgende Remission (gemessen gegen MgO-Weißstandard in einem Spektralphotometer mit nachgeschalteter Ulbricht-Kugel) in der Nähe des oberen umlaufenden Randes etwa in der Mitte einer vertikalen Außenseite des Kastens gekennzeichnet:

| Art des Kunststoffes | Farb-pigment | Farbe der Flaschen-kästen | Remission gegen MgO-Weißstandard | | |
| --- | --- | --- | --- | --- | --- |
| | | | im Spektral-bereich | des neuen Kastens | des gealterten Kastens |
| lineares Polyethylen (HDPE) | Cadmium-Selenid (CdSe) | rot | 400—500 nm | 4—6% | 8—12% |
| | Cadmium-Sulfid (CdS) | gelb | 400—500 nm | 6—10% | 17—20% |
| | Chromoxid ($Cr_2O_3$) | grün | 400—500 nm | 5—14% | 10—15% |
| | Kupfer-Phthalo-cyanin | blau | 550—650 nm | 6—9% | 10—18% |
| isotaktisches Polypropylen | Cadmium-sulfid (CdS) | gelb | 400—500 nm | 5—9% | 25—28% |

Für mit anderen Pigmenten gefärbte Flaschentransportkästen oder solche aus anderen Kunststoffen oder für Kunststoffgegenstände mit anderen Verwendungszwecken lassen sich für ausgewählte Spektralbereiche ebenfalls Sollbereiche angeben, in denen die Remission für einen gealterten Kunststoffgegenstand liegen muß, bevor der Gegenstand auszuscheiden ist.

Beispiel
Für die Erkennung und Ausscheidung gealterter nicht mehr gebrauchstüchtiger Mehrweg-Flaschentransportkästen aus Polyethylen, die mit Cadmium-Selenid dunkelrot in der Masse gefärbt sind, geht man beispielhaft wie folgt vor.

3

**0 068 086**

Nach dem Waschvorgang durchlaufen die gereinigten, trockenen Flaschentransportkästen auf dem Transportband zur Kastenfüllstation die Meßzone. Diese besteht aus einer über dem Transportband innerhalb einer Abschirmung angebrachten Beleuchtungseinrichtung mit weißen Leuchtstoffröhren als Lichtquelle. Die Abschirmung hält unerwünschte Lichteinwirkungen fern. Die Stromversorgung der Leuchtstoffröhren ist stabilisiert. Die Leuchtstoffröhren beleuchten bevorzugt den oberen umlaufenden Rand der Flaschenkästen in gegen die Flächennormale etwas geneigter Richtung. ·

Als Farbmeßgerät wird ein handelsübliches Gerät mit Farb-Videokamera benutzt. Die Achse des Remissionsstrahlenganges steht senkrecht auf der Meßfläche am Flaschenkasten. Die Meßfläche liegt auf einer vertikalen Außenseite der Flaschenkästen etwa in der Mitte des oberen umlaufenden U-Randes. Vor die Videokamera der Farbmeßeinrichtung wird ein Blaufilter geschaltet.

Jeder der über des Transportband in üblicher Weise laufenden Flaschenkästen durchläuft die Meßzone, wobei die Remission der Meßfläche gemessen wird. Liegt der Remissionswert eines Flaschenkastens außerhalb des Sollbereichs, wird der fragliche Kasten nach Verlassen der Meßzone automatisch vom Transportband abgeschoben.

Da für dieses Verfahren eine Relativmessung hinreichend ist, wird die Farbmeßeinrichtung folgendermaßen eingestellt:

Das Anzeigegerät der Farbmeßeinrichtung wird mittels eines unbenutzten Flaschenkastens in der Meßzone durch apparativen Abgleich auf Null eingestellt. Danach wird ein Flaschenkasten gleicher Art, der nicht mehr gebrauchstüchtig ist, in die Meßzone geschoben. Hierbei zeigt das Anzeigegerät der Farbmeßeinrichtung beispielsweise 2,3 mV an. Die Vorrichtung zum Abschieben der Flaschenkästen mit einer außerhalb des Sollbereichs liegenden Remission der Meßfläche wird durch die Farbmeßeinrichtung automatisch ausgelöst, sobalt das Anzeigegerät mehr als 2,3 mV anzeigt.

Der Nullabgleich des Anzeigegerätes und die Einstellung der automatischen Auslösung der Abschiebevorrichtung kann mittels des unbenutzten und des nicht mehr gebrauchstüchtigen Flaschenkastens bei Bedarf beliebig wiederholt werden.

Die Remission des für die Einstellung der Farbmeßeinrichtung benutzten, nicht mehr gebrauchstüchtigen Flaschentransportkastens wurde vorher auf einem Spektralphotometer mit nachgeschalteter Ulbricht-Kugel gegen MgO-Weißstandard gemessen. Die Meßfläche an der kritischen Stelle dieses Flaschenkastens hatte dabei eine mittlere Remission von 10% in Spektralbereich 400—500 nm.

## Patentansprüche

1. Verfahren zur Feststellung des Alterungszustandes von in der Masse gefärbten Kunststoffgegenständen mittels einer zerstörungsfreien berührungslosen Meßmethode zwecks Ausscheiden nicht mehr gebrauchstüchtiger Gegenstände aus einer Vielzahl gleicher Gegenstände, gekennzeichnet durch

— Beleuchten der Kunststoffgegenstände mit einer Lichtquelle, die Licht im gesamten sichtbaren Spektralbereich aussendet,
— Messen der Remission an mindestens einer kritischen Stelle der Oberfläche der Kunststoffgegenstände in einem auf die Farbe der Gegenstände abgestimmten Bereich des Spektrums und
— Ausscheiden aller Kunststoffgegenstände, deren Remission außerhalb eines vorgegebenen Sollbereichs liegt.

2. Verfahren nach Anspruch 1, gekennzeichnet durch Messen der Remission in einem Spektralbereich, der außerhalb des Bereiches der Farbe des Kunststoffgegenstandes liegt.

3. Verfahren nach Anspruch 1 oder 2, gekennzeichnet durch Messen der Remission an zwei kritischen Stellen der Oberfläche der Kunststoffgegenstände, die auf zwei etwa senkrecht aufeinander stehenden Seiten eines Gegenstandes liegen, die bei bestimmungsgemäßem Einsatz des Gegenstandes der Alterung durch Lichteinwirkung ausgesetzt sind.

4. Verfahren nach Anspruch 3, angewendet auf Mehrweg-Flaschentransportkästen gekennzeichnet durch Messen der Remission mehrerer Meßfelder im oberen Teil zweier vertikaler Außenseiten.

5. Verfahren nach Anspruch 4, angewendet auf Mehrweg-Flaschentransportkästen aus Polyethylen, die mit CdSe dunkelrot eingefärbt sind, gekennzeichnet durch

— Messen der Remission im Spektralbereich von 400 bis 500 nm und
— Ausscheiden aller Kästen, deren Remission, gemessen gegen MgO, mehr als 10% beträgt.

## Revendications

1. Procédé pour la détermination de l'état de vieillissement d'objets en matière synthétique colorés dans la masse, au moyen d'une méthode de mesure non destructive et sans contact, en vue d'éliminer, parmi de multiples objets semblables, des objets qui ne sont plus propres à l'usage, caractérisé par le fait

4

— que l'on éclaire les objets en matière synthétique avec une source lumineuse qui émet de la lumière dans tout le domaine spectral visible,
— que l'on mesure la luminance à au moins un endroit critique de la surface des objets en matière synthétique, dans un domaine du spectre accordé sur la couleur des objets et
— que l'on élimine tous les objets en matière synthétique dont la luminance se situe en dehors d'une gamme de consigne prescrite.

2. Procédé selon la revendication 1, caractérisé par le fait que l'on mesure la luminance dans un domaine spectral qui se situe hors du domaine de la couleur de l'objet en matière synthétique.

3. Procédé selon la revendication 1 ou 2, caractérisé par le fait que l'on mesure la luminance en deux endroits critiques de la surface des objets en matière synthétique, qui se trouvent sur deux côtés d'un objet qui sont sensiblement orthogonaux et qui, lors de l'utilisation de l'objet conformément à sa destination, sont exposés au vieillissement par l'action de la lumière.

4. Procédé selon la revendication 3, appliqué à des caisses réutilisables de transport de bouteilles, caractérisé par le fait que l'on mesure la luminance de plusieurs zones de mesure dans la partie verticale de deux côtés extérieurs verticaux.

5. Procédé selon la revendication 4, appliqué à des caisses réutilisables de transport de bouteilles, qui sont réalisées en polyéthylène et sont teintées en rouge sombre par CdSe, caractérisé par le fait

— que l'on mesure la luminance dans le domaine spectral de 400 à 500 nm et
— que l'on élimine toutes les caisses dont la luminance, mesurée relativement à MgO, est supérieure à 10%.

**Claims**

1. A process for determining the ageing condition of mass-dyed plastics articles by means of a non-destructive non-contact measuring method with the objective of excluding from a multiplicity of similar articles those which are no longer fit for use, characterized by

— illumination of the plastics articles using a light source which emits light over the entire visible spectrum,
— measurement of the reflectance at at least one critical position of the surface of the plastics articles in a range of the spectrum which is coordinated with the colour of the articles and
— elimination of all those plastics articles of which the reflectance is outside a predetermined theoretical range.

2. A process according to claim 1, characterized by measurement of the reflectance in a range of the spectrum which is outside the range of the colour of the plastics articles.

3. A process according to claim 1 or 2, characterized by measurement of the reflectance at two critical positions of the surface of the plastics articles which are situated on two approximately mutually perpendicular sides of the articles which are exposed to ageing as a result of the action of light when the articles are used in accordance with their intended purpose.

4. A process according to claim 3, applied to reusable bottle transport crates, characterized by measurement of the reflectance of a plurality of measurement fields in the upper part of two vertical outer sides.

5. A process according to claim 4, applied to reusable bottle transport crates of polyethylene, which are dyed dark red with CdSe, characterized by

— measurement of the reflectance in the spectral range from 400 to 500 nm and
— elimination of all crates, the reflectance of which, measured against MgO, amounts to more than 10%.